(19) Europäisches Patentamt
European Patent Office
Office européen des brevets

(11) Publication number: 0 232 544 A2

(12) # EUROPEAN PATENT APPLICATION

(21) Application number: 86118034.7

(22) Date of filing: 24.12.86

(51) Int. Cl.⁴: C12N 15/00 , C12N 9/64 , C12N 9/72

(30) Priority: 25.12.85 JP 290325/85

(43) Date of publication of application: 19.08.87 Bulletin 87/34

(84) Designated Contracting States: BE CH DE ES FR GB LI NL SE

(71) Applicant: GREEN CROSS CORPORATION
15-1, Imabashi 1-chome Higashi-ku
Osaka-shi
Osaka(JP)

(72) Inventor: Kaneda, Teruo
5-51-105, Nagisa Nishi 1-chome
Hirakata-shi Osaka(JP)
Inventor: Okabayashi, Ken
1-6-24, Kuzuha Asahi 1-chome
Hirakata-shi Osaka(JP)
Inventor: Hayasuke, Naofumi
11-5, Minami Kasugaoka 2-chome
Ibaraki-shi Osaka(JP)
Inventor: Hiramatsu, Ryuji
14-1-1008, Kayashimashinwa-cho
Neyagawa-shi Osaka(JP)
Inventor: Nagai, Masanori
10-10, Nishiyama Waki
Yahata-shi Kyoto(JP)
Inventor: Arimura, Hirofumi
18-1-401, Uenozaka 2-chome
Toyonaka-shi Osaka(JP)

(74) Representative: Hansen, Bernd, Dr.rer.nat. et al
Hoffmann, Eitle & Partner Patentanwälte
Arabellastrasse 4
D-8000 München 81(DE)

(54) **Process for producing physiologically active substances.**

(57) A process for producing physiologically active substances, such as a plasminogen activator selected from tPA, urokinase and single-chain pro-urokinase, by recombinant DNA technology is described. In the present process, a recombinant vector DNA having a DNA sequence therein which encodes a human physiologically active substance is transfected to established cells of human kidney origin to obtain transformed cells which produce and secrete the desired physiologically active substance.

EP 0 232 544 A2

# PROCESS FOR PRODUCING PHYSIOLOGICALLY ACTIVE SUBSTANCES

## FIELD OF THE INVENTION

The present invention relates to a process for producing physiologically active substances by recombinant DNA technology. More specifically, the present invention relates to a process for producing physicologically active substances where a recombinant vector is introduced into established cells of human kidney origin to obtain transformed cells which produce and secrete the desired physiologically active substance.

## BACKGROUND OF THE INVENTION

Escherichia coli was first used as a host for the production of desired physiologically active substances by recombinant DNA technology. Other microorganisms such as yeast, Bacillus subtilis, etc. have recently been utilized because of the easiness of the culture thereof and because of the progress of the study on the vector systems thereof. (See Goeddel, D.V., Itakura, K., et al, Proc. Natl. Acad. Sci., U.S.A., 76 , 106 (1979) and Nagata, S., Taira, S.H. and Weissmann, C., Nature, 284, 1316 (1980)).

More recently, expression systems using mammalian cells as a host have been developed so as to produce high molecular weight glycoproteins.

Some mammalian cells which have heretofore been employed include the following:
CHO-$K_1$cell (Chinese hamster ovary cells: ATCC CCL-61);
BHK cell (Baby hamster kidney cells: ATCC CCL-10);
COS-7 cell (CV-1 Origin, SV-40 cells: ATCC CRL-1651); and
Vero cells (Green monkey kidney cells: ATCC CCL-81).

However, when the known mammalian cells, such as CHO-$K_1$ are used, bovine serum is necessary for their growth, and the physiologically active substance produced, such as a single-chain pro-urokinase - (hereinafter referred to as "pro-UK"), is converted into a double-chain one (as described in Kasai S., et al., J. Biol. Chem., 260, 12377, 1985). In addition, the amount of the physiologically active substance produced using such cells is not sufficient and the product is often contaminated with bovine serum proteins.

## SUMMARY OF THE INVENTION

Accordingly, an object of the present invention is to develop a process for producing a physiologically active substance in a human cell which require no or low amount of bovine serum for growth.

Another object of the present invention is to develop a process for producing a physiologically active substance which retains its natural structure and is not contaminated with bovine serum proteins.

The above-described objects of the present invention have been met by a process for producing physiologically active substances by recombinant DNA technology, wherein DNA was transfected to established cells of human kidney origin by the DNA-$CaPO_4$ co-precipitation technique and the cells are transformed with a vector DNA having inserted therein a DNA sequence which encodes a physiologically active substance and the transformed cells are grown so that the desired physiologically active substance is secreted.

## BRIEF DESCRIPTION OF THE DRAWINGS

Fig. 1 shows a sedimentation pattern of cDNA.

Fig. 2 shows restriction maps of cDNA cloned in each of pUK1, pUK3 and pU4.

Fig. 3 shows a flow-sheet of ligation of pUK1 and pU4, where E means EcoR I, H means Hind III and B means BamH I.

Fig. 4 shows a flow-sheet of ligation of $pcDV_1$ and $pL_1$.

Fig. 5 shows a restriction map of pSV-$G_1$.

Fig. 6 shows restriction maps of pSV-$G_1$-preUK and pSV-$G_1$-Neo.

Fig. 7 shows the structure of pSV-$G_1$-preUK in the region near preUK cDNA, where NC means non-coding region, SS means signal sequence, and SJ means splicing junction.

Fig. 8 shows a Western Blot Analysis for the measurement of the molecular weight of urokinase in a culture fluid.

## DETAILED DESCRIPTION OF THE INVENTION

The established cells of human kidney origin employed in the present invention are derived, for example, from a primary culture of diploid cells obtained from a human fetal kidney. The diploid cells are cultured to establish a cell line. The cells to be used are preferably those which are normally capable of producing the desired physiologically active substances. For example, pro-UK-producing kidney cells are most preferably used for the production of pro-UK.

The cells are grown in cell density of $2 \times 10^4$ to $20 \times 10^4$ cells/ml for about 3 days or so. When the cell number has increased to about 3 to 6 times of the initial number thereof, the cells are trypsinized with a trypsin-EDTA solution to heavest the cells and these cells are preferably used in the practice of the present invention.

The culture media for the incubation of the cells can be, for example, serum-free media such as modified Waymouth's medium or Dulbecco's modified MEM medium used, and in particular, the cells of the present invention are cultured preferably in a serum-free medium containing an appropriate amount (or 0.05 to 0.2 w/v%) of a human serum albumin or in a medium containing a low concentration (or 0.05 to 0.2 v/v%) of a serum from another animal such as bovine serum. The culture medium may additionally contain hydrolyzed lactalbumin, transferrin, a variety of amino acids, a variety of fatty acids or hormones such as insulin.

An air gas (comprising 95% of air and 5% of $CO_2$) is preferably introduced into the culture medium at a gas-flow rate of 10 to 500 ml/min., and the temperature during the culture of the cells is preferably 20 to 37°C. The culture solution is preferably changed by a fresh one every 2 to 3 days.

The thus established cells of human kidney origin are used as a host (recipient), and a physiologically active substance-expression vector is transfected to the host cells by DNA-$CaPO_4$ co-precipitation technique to transform the cells.

The physiologically active substance-expressing vector comprises factors controlling gene expression such as an enhancer and early promoter derived from SV-40, a polyadenylation signal, the gene encoding the physiologically active substance. In addition, a promoter derived from other animal viruses or eukaryotic genes can also be used (as described in Gluzman, Y. eds., Eukaryotic Viral Vectors, Cold Spring Harbor Laboratory, 1982).

For the transformation, recipient cells are inoculated at a density of $5 \times 10^5$ to $7 \times 10^5$ cells/100mm-dish, and an expression vector is added thereto in a DNA amount of about 2 to 4 µg/dish. The cells are thus grown under appropriate selective condition and the resulting transformants are selected. The transformants are preferably grown on a culture carrier. The carrier is preferably a positively charged glass, Daklon, Teflon, Nylon, Orlon, etc. The carrier is added generally to the culture medium at a density of about 0.001 to 0.5 g/cm³, preferably 0.01 to 0.20 g/cm³.

The recovery of the physiologically active substance from the culture fluid is carried out, for example, by an appropriate combination of procedures such as centrifugation, concentration under reduced pressure, salting-out, gel-filtration, ion-exchange chromatography and affinity-chromatography.

For example, pro-UK can be recovered in accordance with the following procedures. The culture fluid is first subjected to centrifugation to recover the supernatant. The thus recovered supernatant is thereafter partially purified by ion-exchange chromatography using preferably as the ion-excharge resin, a weak acidic cationic exchanger. For example, a CM-exchanger or Duolite can be used. The resin is adjusted with a buffer to pH of 4.5 to 6.5, more preferably 5 to 6. The previously recovered supernatant is developed so as to be adsorbed on the resin and washed with the buffer. Then the zymogen is eluted with a buffer having a pH value of 7.5 to 9.5, more preferably 8 to 9. Examples of the buffer to be used in this operation is a phosphate buffer or the like. Next, the eluant is highly purified by affinity chromatography. Any of a polyclonal antibody or a monoclonal antibody can be used as the ligand.

As explained in detail in the above, the present invention provides a method for producing physiologically active substances by recombinant DNA technology where established cells of human kidney origin are used as a host, and the method realizes the high production of a variety of physiologically active substances by the culture of a transformed cell in a serum-free culture medium or in a culture medium containing a serum of a low concentration. Further, the method does not cause the denaturation of the products, and therefore, the method of the present invention is industrially significant for the production of high molecular weight glycoproteins.

Physiologically active substances which can be produced by the method of the present invention are, for example, tPA, urokinase, pro-UK, IFN-$\gamma$ , TNF, etc.

The present invention is explained in greater detail by reference to the following example, where one typical embodiment of pro-UK is illustrated concretely. However, this example is not whatsoever intended to be interpreted as limiting the scope of the present invention.

## Example

(I) Isolation and purification of mRNA:

(i) Cells:

In order to extract RNA from urokinase-producing cells, the cells were cultured in Dulbecco's MEM medium supplemented with 5% (v/v) fetal calf serum (FCS). Afterwards, the medium was replaced by Dulbecco's MEM medium supplemented with I% (v/v) FCS and the cells were further cultured therein for I0 to I5 hours at 37°C. $2 \times 10^9$ to $5 \times 10^9$ cells from among those thus cultured were collected by treatment with trypsin, and the total RNA was extracted as described below.

(ii)Recovery of mRNA:

In accordance with the method as illustrated in Japanese Patent Application (OPI) No. I48899/83 (Applicant: Green Cross Corporation), purification of mRNA from the total RNA was carried out to obtain mRNA having a size of I9 to 20S which showed a urokinase-activity when the RNA was injected into Xenopus oocytes.

(2) Synthesis of single-stranded cDNA:

The synthesis of cDNA was carried out according to the method as described in Haruin, P.W. et al, J. Biol. Chem., 253 , 2483-2495 (I978) and Maniatis et al., Molecular cloning, A Laboratory manual, Cold Spring Harbor Laboratory, I982.

More specifically, the reaction mixture as shown in Table I below was heated at 65°C for 5 minutes and then chilled on ice. Thereafter, 20 µg of mRNA was added thereto, and then left on an ice.

Table 1

| Reaction Mixture | Amount | Final Concentration |
|---|---|---|
| 0.5M Tris-HCl (pH 8.3) | 20 μl | 50 mM |
| 1.4M KCl | 10 μl | 70 mM |
| 025M $MgCl_2$ | 8 μl | 10 mM |
| 0.05M dNTPs | each 2 μl | each 0.5 mM |
| 50 Ci $\gamma$-$^{32}$P-dCTP | 10 μl | |
| 0.2M DTT | 10 μl | 10 mM |
| Oligo $(dT)_{12-18}$ (250 μg/ml) | 20 μl | 25 μg/ml |
| mRNA | 10 or 20 μg | 500 to 1000 μg/ml |
| AMV reverse transcriptase (manufactured by Life Science, Inc.) | 160 units | 800 U/ml |
| RNase inhibitor (manufactured by Wako Pure Chemical Industries, Ltd.) | 22 units | 70 U/ml |
| $H_2O$ | to make 200 μl | |

After the incubation at 46°C for l0 to l2 minutes, the single-stranded cDNA was synthesized. Next, 20 μl of 0.5M EDTA (pH 8.0) was added to the reaction mixture to terminate the reaction. The equal volume (200 μl) of a phenol/chloroform (l:l) solution was added to the reaction mixture and voltexed and thereafter centrifuged for 3 minutes at l0,000 rpm. The aqueous phase was then taken out. The 200 μl of Sephadex G-l00 buffer (comprising lmM Tris-HCl (pH 8.0), lmM EDTA and l00mM NaCl) was further added to the lower phenol phase for re-extraction. The two aqueous layers were mixed, and 60 μl of 80%(v/v)-glycerol was added.

The reaction mixture was then applied to Sephadex G-100 column and subjected to elution with G-l00 buffer described above. The eluant was fractionated, five drops in each one fraction and Cherenkov Count was measured for each fraction. The fraction of peak radio activity were collected.

0.1 volume of 3M sodium acetate buffer, pH 5.2, l0 μg of yeast tRNA and 2 volumes of ethanol were added and the mixture was cooled at -80°C for 20 minutes. A precipitate was collected by centrifugation at l5,000 rpm for l0 minutes, and the precipitate was dried under reduced pressure. Then, the precipitate was dissolved in 300 μl of a 0.lN NaOH solution, incubated for 20 minutes at 70°C and then cooled with water. To this solution was added about 30 μl of lN HCl solution for neutralization. Next, l0 μg of tRNA and 2 volumes of ethanol were added to the solution and the precipitate formed was collected by centrifugation at l5,000 rpm for l0 min. to obtain 3.65 μg of single-stranded cDNA.

(3) Synthesis of double-stranded cDNA :

For the synthesis of double-stranded cDNA, 3.65 μg of the single-stranded cDNA was resuspended in l00 μl of the reaction mixture as shown in Table 2 below.

Table 2

| Reaction Mixture Solution | Amount | Final Concentration |
|---|---|---|
| 0.5M HEPES-NaOH (pH 6.9) | 20 µl | 100 mM |
| 67mM $MgCl_2$ | 20 µl | 13.4 mM |
| 0.7M KCl | 20 µl | 140 mM |
| 02M DTT | 5 µl | 10 mM |
| 5mM dNTPs | 20 µl | 1 mM |
| $H_2O$ | to make 100 µl | |

50 units (5 µl) of DNA polymerase I (Klenow fragment) and 90 µl of water were added whereby the total amount of the reaction mixture was 200 µl and incubated overnight at I5°C.

20 µl of a 0.2M EDTA (pH 8.0) was added to the reaction mixture and then, the equal volume (220 µl) of a phenol/chloroform (I:I) solution was added. The resulting solution was subjected to centrifugation. The upper layer was recovered, and the phenol layer was re-extracted with 220 µl of TE-buffer (I0mM Tris-HCl - (pH 8.0) and ImM EDTA). The two aqueous layers were combined and 60 µl of 80% (v/v) glycerol was added thereto.

The resulting solution was applied to a Sephadex G-I00 column and subjected to elution with G-I00 buffer. The eluant was fractionated, five drops in each fraction. Cherenkov count was measured for each fraction and the fractions of peak radio activity were collected. 5 µg of tRNA and 0.I volume of 3M sodium acetate buffer, pH 5.2, were added to the solution, and then ethanol was added thereto for precipitation.

The precipitate was collected by centrifugation at I5,000 rpm for I0 minutes and dried under reduced pressure, to obtain double stranded (ds) cDNA. The yield of the ds cDNA was 5.3 µg.

(4) Prolongation of double-stranded cDNA by the use of reverse transcriptase:

The double-stranded cDNA was then extended by the use of a reverse transcriptase.

More specifically, the obtained ds cDNA was dissolved in 20 µl of water, and further, 30 µl of a reaction mixture as shown in Table 3 below was added thereto and the two were mixed.

Table 3

| Reaction Mixture | Amount |
|---|---|
| 1M Tris-HCl (pH 8.3) | 5 µl |
| 1M KCl | 7 µl |
| 250mM $MgCl_2$ | 2 µl |
| dNTPs (20mM) | 2.5 µl |
| 700mM β-mercaptoethanol | 2 µl |
| AMV reverse transcriptase | 2 µl |
| $H_2O$ | to make 30 µl |

The resulting mixture was incubated for 60 minutes at 42°C and 5 µl of 0.2M EDTA (pH 8.0) was added to terminate the reaction. The reaction mixture was extracted with the equal volume (55 µl) of a phenol/chloroform (l:l) solution and ds cDNA was recovered by ethanol precipitation as mentioned above.

(5) Sl unclease digestion:

The resulting prolonged ds cDNA was digested with Sl nulcease, in accordance with a method by Shenk, T.E., et al., Proc. Natl. Acad. Sci:, USA, 72, 989, (l975).

More specifically, ds cDNA was suspended in l00 µl of Sl nuclease buffer (comprising 0.3M NaCl, 30mM sodium acetate (pH 4.5) and 3mM $ZnCl_2$) and incubated at 37°C for 30 minutes to dissolve the ds cDNA. Then, 0.5 unit of Sl nuclease (manufactured by PL Biochemical Co.) was added thereto and incubated for 30 minutes at 37°C. Then, 2 units of Sl nuclease were further added to the reaction mixture and then further incubated at 37°C for l5 minutes.

Then, 20 µl of a 0.2M EDTA (pH 8.0) was added to the reaction solution to terminate the reaction and the solution was extracted with l20 µl of a phenol/chloroform (l:l) solution.

The phenol layer was re-extracted with the equal volume of TE buffer, and then the two aqueous layers were combined and ethanol was added thereto to obtain ds cDNA precipitate.

(6) Sucrose density-gradient centrifugation treatment of ds cDNA:

The ds cDNA obtained was layered on 5 to 25% (w/v) linear sucrose density-gradient and centrifugation was carried out at 38,000 rpm and for l5 hours at 4°C. Fig. l shows the sedimentation pattern of ds cDNA - (which is designated by o——— o). In addition, the sedimentation pattern of rat globin ds cDNA is shown in Fig. l as a marker (which is designated by Δ ——— Δ). Large cDNA fractions of Fraction No. 6 to l5 were collected (hereinafter "large ds cDNA"). The amount of the large ds cDNA was 722 ng.

(7) Addition of poly-(dC)-tail to ds cDNA:

The average size of the synthesized ds cDNA is assumed to be l,500 bp and a (dC)-tail reaction was carried out in accordance with the method described in Nelson and Brutlag, Methods in Enzymol., 68, 4l-50, l979. In the present experiment, 5 units of a terminal transferase were used and the reaction was carried out at 37°C. As a result, about 20 (dC)-tails were added to the 3′-end of dC DNA.

About l5 poly(dG)-tails were added to the Pst I site of plasmid pBR322 which had been prepared separately.

Next, the poly-(dC)-tailed ds cDNA and poly-(dG)-tailed pBR 322 were mixed at an equimolar ratio and annealing was performed at from 70°C to 37°C.

(8) Transformation:

E. coli strain RRl was transformed by the method described in Lederberg and Cohen, J. Bacteriol, ll9, l072-l074 (l974), to obtain about 70,000 transformants. The ampicillin-sensitivity of the obtained transformants was investigated by means of replica plating. As a result, 90% or more of all the transformants, or about 60,000 colonies were found to be carrying cDNA.

(9) Cloning of the urokinase cDNA:

For the cloning of the urokinase cDNA, the synthetic l4-mer oligonucleotides as deduced from the known amino acid sequence of high molecular weight-type urokinase obtained from human urine (Günzler et al., Hoppe Seyler's Z. Physiol. Chem., 363, ll56-ll65 (l982), Steffeus et al, Hoppe Seyler's Z. Physiol. Chem., 363, l043-l058, l982) was synthesized and this was used as a hybridization probe for the screening of the transformants.

(i) Synthetic oligonucleotides probe:

A 14-base long synthetic oligonucleotides (manufactured by Nippon Zeon Co.) was used as a probe (hereinafter referred to as "probe").

Table 4 below shows the base sequence of the probe used and the amino acid sequence of the corresponding urokinase. The probe used correspond to the amino acids 73 through 77 of a B-chain of high molecular weight-type urokinase. As apparent from Table 4, the probe is a mixture comprising eight kinds of oligonucleotides.

## Table 4

Oligonucleotide probe for isolation of human urokinase cDNA

Probe

| | | | | | | | | | | | |
|---|---|---|---|---|---|---|---|---|---|---|---|
| | | | 73 | | 74 | | 75 | | 76 | | 77 | | |
| Amino acid sequence | $NH_2$ | - | Glu | - | Met | - | Lys | - | Phe | - | Glu | - | COOH |
| Codon | 5' | - | $GA^{G}_{A}$ | - | AUG | - | $AA^{G}_{A}$ | - | $UU^{U}_{C}$ | - | $GA^{G}_{A}$ | - | 3' |

14-mer Mixture

| | | | | | | | | | | | | | |
|---|---|---|---|---|---|---|---|---|---|---|---|---|---|
| Codon | 3' | - | $CT^{T}_{C}$ | - | TAC | - | $TT^{T}_{C}$ | - | $AA^{G}_{A}$ | - | CT | - | 5' |

In the middle of Table 4, the possible and complete sequence of mRNA coresponding to the amino acid sequence is shown. In the lowermost part of Table 4, the base sequence of probe DNA which is complimentary to this mRNA is shown. The third base in the amino acid 77 (Glu) codon of the probe is not contained in the probe DNA.

(ii) 5'-end labeling of the probe DNA

(a) $^{32}$p-labeling of probe 5'-end:

The probe was synthesized in the form having 5'-OH, and the 5'-end was labeled with $\gamma$-$^{32}$P ATP - (Amersham, PBI0218) and T4 polynucleotide kinase.

More specifically, the molar ratio of $^{32}$P-ATP and 5'-OH end was 5:l and the reaction was carried out in the reaction solution described below.

Reaction Solution:

| | |
|---|---|
| Probe DNA | 6 to 80 pmole of 5'-OH end |
| (10X) kinase buffer (*1) | 2 µl |
| T4 polynucleotide kinase | 1 µl |
| $\gamma$-$^{32}$P ATP | 5 moles of 5'-OH end (*2) |
| $H_2O$ | to make 20 µl |

Note:

(*1) (10X) kinase buffer:

0.7M Tris HCl (pH 7.6)

0.1M $MgCl_2$

50mM DTT

(*2) The molar amount of $\gamma$-$^{32}$P ATP was calculated on the basis of 5,000 Ci/mmole.

The reaction was carried out for 60 to 120 minutes at 37°C. After the completion of the reaction, 1 µl of a 0.5M EDTA (pH 8.0), 35 µl of 1M sodium chloride (in 10mM Tris-HCl (pH 7.2) and 1mM-EDTA) and 10.5 µl of a tRNA solution (1mg/ml) were added to the reaction mixture whereby the final amount of the reaction mixture was 35 µl. Thus, the reaction solution was then heat-treated at 70°C for 3 minutes to inactivate the polynucleotide kinase.

(b) Purification of $^{32}$P-labeled probe:

The reaction mixture as prepared as described above was subjected to chromatography on a NACS-52 mini-column (BRL) for the purification of the $^{32}$P-labeled probe.

The specific activity of the thus purified probe DNA was about $1.0 \times 10^9$ dpm/µg-DNA.

(iii) Colony hybridization:

The probe prepared as described above was used and colony hybridization was carried out in accordance with the method of Maniatis et al, Molecular Cloning, A Laboratory Manual, Cold Spring Harbor Laboratory (1982), whereby the urokinase cDNA was screened.

From among the above-mentioned about 70,000 transformants, about 10,000 transformants were subjected to colony hybridization by the use of the [$\gamma$-$^{32}$P]-labeled synthetic probe. As a result, 65 colonies, which were considered relatively strongly hybridized, were screened out. These 65 colonies were re-screened with the same probe. As a result, four colonies were found to hybridized extremely strongly with the probe DNA. From the thus screened four colonies were prepared plasmid DNA (each of which was named pUK1, 2, 3 and 4, respectively) in accordance with the method as described in Birnboim and Doly, Nucleic Acid Res., 7, 1513, 1979, and thus, the size of each cDNA was determined. pUK1 had the largest cDNA among the four colonies, i.e., about 1,900 bp. pUK3 and pUK4 are smaller, i.e. about 1,300 bp and about 1,150 bp, resectively. pUK2 had the smallest cDNA, i.e., about 170 bp.

(10) Formation of restriction enzyme map:

A restriction map of cDNA cloned in each of pUKl, pUK3 and pUK4 was formed and compared with one another.

The restriction enzymes used in the formation of the maps were Acc I, Atu I, Ava I, Ava II, BamH I, Bcl I, Bgl I, Bgl II, BstE II, Cla I, EcoR I, Fnu4H I, HaeIII, Hinc II, Hind III, Kpn I, Nco I, Mlu I, Pst I, Pvu II, Rsu I, Sac I, Sal I, Sau96 I, Sca I, Sma I, Stu I, Xba I and Xho I, and the buffer for each restriction enzyme used in the reaction is given in Table 5 below.

It should be noted that Ava I used the Rsa I-buffer; Sac I used the Kpn I-buffer; Stu I, Sal I and Xho I used the BamH I-buffer; Nco I and Sca I used the Bgl I-buffer; and Mlu I used the Hin d III-buffer.

Table 5

Final concentration of each component

| Enzyme | Tris-HCl (mM) | pH | NaCl (mM) | $MgCl_2$ (mM) | $(NH_4)_2SO_4$ (mM) | KCl (mM) | mercapto-ethanol (mM) |
|---|---|---|---|---|---|---|---|
| ACC I | 10 | 7.5 | 60 | 7 | | | 5 |
| Alu I | 6 | 7.6 | 50 | 6 | | | 5 |
| Ava II | 6 | 8.0 | 60 | 10 | | | 5 |
| BamH I | 10 | 8.0 | 100 | 7 | | | 5 |
| Bcl I | 6 | 7.4 | | 10 | | 75 | - |
| Bgl I | 10 | 7.4 | 66 | 10 | | | 2 |
| Bgl II | 10 | 7.5 | 100 | 10 | | | 5 |
| BstE II | 6 | 7.9 | 150 | 6 | | | - |
| Cla I | 6 | 7.9 | 50 | 6 | | | 5 |
| EcoR I | 100 | 7.5 | 50 | 7 | | | 5 |
| Fnu4H I | 6 | 7.4 | 6 | 6 | | 5 | 5 |
| Hae III | 10 | 7.5 | 60 | 7 | | | 5 |
| Hinc II | 10 | 8.0 | 60 | 7 | | | 5 |
| Hind III | 10 | 7.5 | 60 | 7 | | | 5 |
| Kpn I | 6 | 7.5 | 6 | 6 | | | - |
| Pst I | 20 | 7.5 | | 10 | 50 | | - |
| Pvu II | 6 | 7.5 | 60 | 6 | | | - |
| Rsa I | 6 | 8.0 | 50 | 12 | | | 12 |
| Sau96 I | 6 | 7.4 | 60 | 15 | | | 5 |
| Sma I | 6 | 8.0 | | 6 | | 20 | 5 |
| Xba I | 6 | 7.9 | 50 | 6 | | | |

Each plasmid was digested with the corresponding restriction enzyme, and then restriction map of each plasmid was determined. When the plasmid was cleaved with EcoR I, a fragment having the same size (420 bp) was obtained from both plasmids pUKl and pUK4, and after the double-digestion with BamH I and EcoR I as well as BamH I and Pst I, a fragment having the same size was obtained from both plasmids pUKl and pUK3. The positions of the restriction map of the cDNA of pUKl, pUK3 and pUK4 were found to be the same as shown in Fig. 2. Thus, the cDNA inserted into pUKl, pUK3 and pUK4 was considered to be the same. Next, the DNA base sequence of a part of pUKl and pUK4 was determined, and the amino acid sequence was investigated.

(ll) Determination of sequence of urokinase cDNA:

The DNA sequence and the amino acid sequence of the urokinase cDNA were determined in accordance with the method as described in Maxam-Gilbert, Proc. Natl. Acad. Sci., USA, 74, 560, l977. (See the following "Sequence I".) The possible amino acid sequence corresponding to the determined cDNA sequence was compared with the amino acid sequence of the known human urinary urokinase. As a result, it was confirmed that the present cDNA encodes a prepro-urokinase which comprises 43l amino acids and includes a signal peptide composed of 20 amino acids.

(l2) Amino acid sequence of urokinase:

The determination as to whether or not the present cDNA is the same as urokinase cDNA was carried out by obtaining the amino acid sequence from the DNA sequence of the present cDNA as determined - (Sequence I) and then comparing the thus obtained amino acid sequence with the amino acid sequence of the known human urinary urokinase.

The following Sequence II shows the known total amino acid sequence of the A-and B-chains of high molecular weight-type urokinase obtain from human urine.

Sequence I: Base-sequence and amino acid-sequence of pre-structural gene, signal sequence and non-coding region

5'TCCACCTGTCCCCGCAGCGCCGGCTCGCGCCCTCCTGCCGCAGCCACCGAGCCGCCGTCTAGCGCCCCGACCTCGCCACC

```
-20                                     -10                                      -1
Met Arg Ala Leu Leu Ala Arg Leu Leu Leu Cys Val Leu Val Val Ser Asp Ser Lys Gly
ATC AGA GCC CTG CTG GCG CGC CTG CTT CTC TGC GTC CTG GTC GTG AGC GAC TCC AAA GGC

  1                                      10                                      20
Ser-Asn-Glu-Leu-His-Gln-Val-Pro-Ser-Asn-Cys-Asp-Cys-Leu-Asn-Gly-Gly-Thr-Cys-Val-
AGC AAT GAA CTT CAT CAA GTT CCA TCG AAC TGT GAC TGT CTA AAT GGA GGA ACA TGT GTG

 21                                      30                                      40
Ser-Asn-Lys-Tyr-Phe-Ser-Asn-Ile-His-Trp-Cys-Asn-Cys-Pro-Lys-Lys-Phe-Gly-Gly-Gln-
TCC AAC AAG TAC TTC TCC AAC ATT CAC TGG TGC AAC TGC CCA AAG.AAA TTC GGA GGG CAG

 41                                      50                                      60
His-Cys-Glu-Ile-Asp-Lys-Ser-Lys-Thr-Cys-Tyr-Glu-Gly-Asn-Gly-His-Phe-Tyr-Arg-Gly-
CAC TGT GAA ATA GAT AAG TCA AAA ACC TGC TAT GAG GGG AAT GGT CAC TTT TAC CGA GGA

 61                                      70                                      80
Lys-Ala-Ser-Thr-Asp-Thr-Met-Gly-Arg-Pro-Cys-Leu-Pro-Trp-Asn-Ser-Ala-Thr-Val-Leu-
AAG GCC AGC ACT GAC ACC ATG GGC CGG CCC TGC CTG CCC TGG AAC TCT GCC ACT GTC CTT

 81                                      90                                     100
Gln-Gln-Thr-Tyr-His-Ala-His-Arg-Ser-Asp-Ala-Leu-Gln-Leu-Gly-Leu-Gly-Lys-His-Asn-
CAG CAA ACG TAC CAT GCC CAC AGA TCT GAT GCT CTT CAG CTG GGC CTG GGG AAA CAT AAT

101                                      10                                     120
Tyr-Cys-Arg-Asn-Pro-Asp-Asn-Arg-Arg-Arg-Pro-Trp-Cys-Tyr-Val-Gln-Val-Gly-Leu-Lys-
TAC TGC AGG AAC CCA GAC AAC CGG AGG CGA CCC TGG TGC TAT GTG CAG GTG GGC CTA AAG
```

```
121                                130                                140
Pro-Leu-Val-Gln-Glu-Cys-Met-Val-His-Asp-Cys-Ala-Asp-Gly-Lys-Lys-Pro-Ser-Ser-Pro-
CCG CTT GTC CAA GAG TGC ATG GTG CAT GAC TGC GCA GAT GGA AAA AAG CCC TCC TCT CCT

141                                150                                160
Pro-Glu-Glu-Leu-Lys-Phe-Gln-Cys-Gly-Gln-Lys-Thr-Leu-Arg-Pro-Arg-Phe-Lys-Ile-Ile-
CCA GAA GAA TTA AAA TTT CAG TGT GGC CAA AAG ACT CTG AGG CCC CGC TTT AAG ATT ATT

161                                170                                180
Gly-Gly-Glu-Phe-Thr-Thr-Ile-Glu-Asn-Gln-Pro-Trp-Phe-Ala-Ala-Ile-Tyr-Arg-Arg-His-
GGG GGA GAA TTC ACC ACC ATC GAG AAC CAG CCC TGG TTT GCG GCC ATC TAC AGG AGG CAC

181                                190                                200
Arg-Gly-Gly-Ser-Val-Thr-Tyr-Val-Cys-Gly-Gly-Ser-Leu-Ile-Ser-Pro-Cys-Trp-Val-Ile-
CGG GGG GGC TCT GTC ACC TAC GTG TGT GGA GGC AGC CTC ATC AGC CCT TGC TGG GTG ATC

201                                210                                220
Ser-Ala-Thr-His-Cys-Phe-Ile-Asp-Tyr-Pro-Lys-Lys-Glu-Asp-Tyr-Ile-Val-Tyr-Leu-Gly-
AGC GCC ACA CAC TGC TTC ATT GAT TAC CCA AAG AAG GAG GAC TAC ATC GTC TAC CTG GGT

221                                230                                240
Arg-Ser-Arg-Leu-Asn-Ser-Asn-Thr-Gln-Gly-Glu-Met-Lys-Phe-Glu-Val-Glu-Asn-Leu-Ile-
CGC TCA AGG CTT AAC TCC AAC ACG CAA GGG GAG ATG AAG TTT GAG GTG GAA AAC CTC ATC

241                                250                                260
Leu-His-Lys-Asp-Tyr-Ser-Ala-Asp-Thr-Leu-ala-His-His-Asn-Asp-Ile-Ala-Leu-Leu-Lys-
CTA CAC AAG GAC TAC AGC GCT GAC ACG CTT GCT CAC CAC AAC GAC ATT GCC TTG CTG AAG

261                                270                                280
Ile-Arg-Ser-Lys-Glu-Gly-Arg-Cys-Ala-Gln-Pro-Ser-Arg-Thr-Ile-Gln-Thr-Ile-Cys-Leu-
ATC CGT TCC AAG GAG GGC AGG TGT GCG CAG CCA TCC CGG ACT ATA CAG ACC ATC TGC CTG

281                                290                                300
Pro-Ser-Met-Tyr-Asn-Asp-Pro-Gln-Phe-Gly-Thr-Ser-Cys-Glu-Ile-Thr-Gly-Phe-Gly-Lys-
CCC TCG ATG TAT AAC GAT CCC CAG TTT GGC ACA AGC TGT GAG ATC ACT GGC TTT GGA AAA
```

```
301                                        310                                        320
Glu-Asn-Ser-Thr-Asp-Tyr-Leu-Tyr-Pro-Glu-Gln-Leu-Lys-Met-Thr-Val-Val-Lys-Leu-Ile-
GAG AAT TCT ACC GAC TAT CTC TAT CCG GAG CAG CTG AAG ATG ACT GTT GTG AAG CTG ATT

321                                        330                                        340
Ser-His-Arg-Glu-Cys-Gln-Gln-Pro-His-Tyr-Tyr-Gly-Ser-Glu-Val-Thr-Thr-Lys-Met-Leu-
TCC CAC CGG GAG TGT CAG CAG CCC CAC TAC TAC GGC TCT GAA GTC ACC ACC AAA ATG CTG

341                                        350                                        360
Cys-Ala-Ala-Asp-Pro-Gln-Trp-Lys-Thr-Asp-Ser-Cys-Gln-Gly-Asp-Ser-Gly-Gly-Pro-Leu-
TGT GCT GCT GAC CCA CAG TGG AAA ACA GAT TCC TGC CAG GGA GAC TCA GGG GGA CCC CTC

361                                        370                                        380
Val-Cys-Ser-Leu-Gln-Gly-Arg-Met-Thr-Leu-Thr-Gly-Ile-Val-Ser-Trp-Gly-Arg-Gly-Cys-
GTC TGT TCC CTC CAA GGC CGC ATG ACT TTG ACT GGA ATT GTG AGC TGG GGC CGT GGA TGT

381                                        390                                        400
Ala-Leu-Lys-Asp-Lys-Pro-Gly-Val-Tyr-Thr-Arg-Val-Ser-His-Phe-Leu-Pro-Trp-Ile-Arg
GCC CTG AAG GAC AAG CCA GGC GTC TAC ACG AGA GTC TCA CAC TTC TTA CCC TGG ATC CGC

401
Ser-His-Thr-Lys-Glu-Glu-Asn-Gly-Leu-Ala-Leu-Stop
AGT CAC ACC AAG GAA GAG AAT GGC CTG GCC CTC TGA GGGTCCCCAGGGAGGAAACGGGCACCACCCGC

TTTCTTGCTGGTTGTCATTTTTGCAGTAGAGTCATCTCCATCAGCTGTAAGAAGAGACTGGGAAGAT  3'
```

Sequence II

A-Chain

```
 1                                      10                                      20
Ser-Asn-Glu-Leu-His-Gln-Val-Pro-Ser-Asn-Cys-Asp-Cys-Leu-Asn-Gly-Gly-Thr-Cys-Val-

 21                                     30                                      40
Ser-Asn-Lys-Tyr-Phe-Ser-Asn-Ile-His-Trp-Cys-Asn-Cys-Pro-Lys-Lys-Phe-Gly-Gly-Gln-

 41                                     50                                      60
His-Cys-Glu-Ile-Asp-Lys-Ser-Lys-Thr-Cys-Tyr-Glu-Gly-Asn-Gly-His-Phe-Tyr-Arg-Gly-

 61                                     70                                      80
Lys-Ala-Ser-Thr-Asp-Thr-Met-Gly-Arg-Pro-Cys-Leu-Pro-Trp-Asn-Ser-Ala-Thr-Val-Leu-

 81                                     90                                     100
Gln-Gln-Thr-Tyr-His-Ala-His-Arg-Ser-Asp-Ala-Leu-Gln-Leu-Gly-Leu-Gly-Lys-His-Asn-

101                                    110                                     120
Tyr-Cys-Arg-Asn-Pro-Asp-Asn-Arg-Arg-Arg-Pro-Trp-Cys-Tyr-Val-Gln-Val-Gly-Leu-Lys

121                                    130                                     140
Pro-Leu-Val-Gln-Glu-Cys-Met-Val-His-Asp-Cys-Ala-Asp-Gly-Lys-Lys-Pro-Ser-Ser-Pro-

141                                    150
Pro-Glu-Glu-Leu-Lys-Phe-Gln-Cys-Gly-Gln-Lys-Thr-Leu-Arg-Pro-Arg-Phe- * * * * *
```

B-Chain

```
 1                                          10                                      20
Ile-Ile-Gly-Gly-Glu-Phe-Thr-Thr-Ile-Glu-Asn-Gln-Pro-Trp-Phe-Ala-Ala-Ile-Tyr-Arg-

21                                          30                                      40
Arg-His-Arg-Gly-Gly-Ser-Val-Thr-Tyr-Val-Cys-Gly-Gly-Ser-Leu-Ile-Ser-Pro-Cys-Trp-

41                                          50                                      60
Val-Ile-Ser-Ala-Thr-His-Cys-Phe-Ile-Asp-Tyr-Pro-Lys-Lys-Glu-Asp-Tyr-Ile-Val-Tyr-

61                                          70                                      80
Leu-Gly-Arg-Ser-Arg-Leu-Asn-Ser-Asn-Thr-Gln-Gly-Glu-Met-Lys-Phe-Glu-Val-Glu-Asn-

81                                          90                                     100
Leu-Ile-Leu-His-Lys-Asp-Tyr-ser-Ala-Asp-Thr-Leu-Ala-His-His-Asn-Asp-Ile-Ala-Leu-

101                                        110                                     120
Leu-Lys-Ile-Arg-Ser-Lys-Glu-Gly-Arg-Cys-Ala-Gln-Pro-Ser-Arg-Thr-Ile-Gln-Thr-Ile-

121                                        130                                     140
Cys-Leu-Pro-Ser-Met-Tyr-Asn-Asp-Pro-Gln-Phe-Gly-Thr-Ser-Cys-Glu-Ile-Thr-Gly-Phe-

141                                        150                                     160
Gly-Lys-Glu-Asn-Ser-Thr-Asp-Tyr-Leu-Tyr-Pro-Glu-Gln-Leu-Lys-Met-Thr-Val-Val-Lys-

161                                        170                                     180
Leu-Ile-Ser-His-Arg-Glu-Cys-Gln-Gln-Pro-His-Tyr-Tyr-Gly-Ser-Glu-Val-Thr-Thr-Lys-

181                                        190                                     200
Met-Leu-Cys-Ala-Ala-Asp-Pro-Gln-Trp-Lys-Thr-Asp-Ser-Cys-Gln-Gly-Asp-Ser-Gly-Gly-

201                                        210                                     220
Pro-Leu-Val-Cys-Ser-Leu-Gln-Gly-Arg-Met-Thr-Leu-Thr-Gly-Ile-Val-Ser-Trp-Gly-Arg-
```

221 230 340
Gly-Cys-Ala-Leu-Lys-Asp-Lys-Pro-Gly-Val-Tyr-Thr-Arg-Val-Ser-His-Phe-Leu-Pro-Trp-

241 250
Ile-Arg-Ser-His-Thr-Lys-Glu-Glu-Asn-Gly-Leu-Ala-Leu

The cDNA base sequence as obtained by the above-mentioned sequencing was compared with the amino acid sequence (Sequence II) of urokinase obtained from human urine, and as a result, it was confirmed that both pUKI and pUK4 contained a portion of urokinase precursor cDNA.

The following Sequence III shows the complete base sequence from the 5′-end to the 3′-non-coding region of cDNA encoding the urokinase amino acid sequence as formed on the basis of the result of the equencing of each fragment.

Sequence III

```
5'TCCACCTGTCCCCGCAGCGCCGGCTCGCGCCCTCCTGCCGCAGCCACCGAGCCGCCGTCTAGCGCCCCGACCTCGCCACC

 -20                                     -10                                    -1
 Met Arg Ala Leu Leu Ala Arg Leu leu Leu Cys Val Leu Val Val Ser Asp Ser Lys Gly
 ATG AGA GCC CTG CTG GCG CGC CTG CTT CTC TGC GTC CTG GTC GTG AGC GAC TCC AAA GGC

 +1       10        20        30        40        50        60        70        80
 AGCAATGAACTTCATCAAGTTCCATCGAACTGTGACTGTCTAAATGGAGGAACATGTGTGTCCAACAAGTACTTCTCCAA
 Ser                Mature UROKINASE

 81       90       100       110       120       130       140       150       160
 CATTCACTGGTGCAACTGCCCAAAGAAATTCGGAGGGCAGCACTGTGAAATAGATAAGTCAAAAACCTGCTATGAGGGGA

161      170       180       190       200       210       220       230       240
 ATGGTCACTTTTACCGAGGAAAGGCCAGCACTGACACCATGGGCCGGCCCTGCCTGCCCTGGAACTCTGCCACTGTCCTT

241      250       260       270       280       290       300       310       320
 CAGCAAACGTACCATGCCCACAGATCTGATGCTCTTCAGCTGGGCCTGGGGAAACATAATTACTGCAGGAACCCAGACAA

321      330       340       350       360       370       380       390       400
 CCGGAGGCGACCCTGGTGCTATGTGCAGGTGGGCCTAAAGCCGCTTGTCCAAGAGTGCATGGTGCATGACTGCGCAGATG

401      410       420       430       440       450       460       470       480
 GAAAAAAGCCCTCCTCTCCTCCAGAAGAATTAAAATTTCAGTGTGGCCAAAAGACTCTGAGGCCCCGCTTTAAGATTATT

481      490       500       510       520       530       540       550       560
 GGGGGAGAATTCACCACCATCGAGAACCAGCCCTGGTTTGCGGCCATCTACAGGAGGCACCGGGGGGGCTCTGTCACCTA

561      570       580       590       600       610       620       630       640
 CGTGTGTGGAGGCAGCCTCATCAGCCCTTGCTGGGTGATCAGCGCCACACACTGCTTCATTGATTACCCAAAGAAGGAGG

641      650       660       670       680       690       700       710       720
 ACTACATCGTCTACCTGGGTCGCTCAAGGCTTAACTCCAACACGCAAGGGGAGATGAAGTTTGAGGTGGAAAACCTCATC
```

```
721       730       740       750       760       770       780       790       800
 CTACACAAGGACTACAGCGCTGACACGCTTGCTCACCACAACGACATTGCCTTGCTGAAGATCCGTTCCAAGGAGGGCAG

801       810       820       830       840       850       860       860       880
 GTGTGCGCAGCCATCCCGGACTATACAGACCATCTGCCTGCCCTCGATGTATAACGATCCCCAGTTTGGCACAAGCTGTG

881       890       900       910       920       930       940       950       960
 AGATCACTGGCTTTGGAAAAGAGAATTCTACCGACTATCTCTATCCGGAGCAGCTGAAGATGACTGTTGTGAAGCTGATT

961       970       980       990      1000      1010      1020      1030      1040
 TCCCACCGGGAGTGTCAGCAGCCCCACTACTACGGCTCTGAAGTCACCACCAAAATGCTGTGTGCTGCTGACCCACAGTG

1041     1050      1060      1070      1080      1090      1100      1110      1120
 GAAAACAGATTCCTGCCAGGGAGACTCAGGGGGACCCCTCGTCTGTTCCCTCCAAGGCCGCATGACTTTGACTGGAATTG

1121     1130      1140      1150      1160      1170      1180      1190      1200
 TGAGCTGGGGCCGTGGATGTGCCCTGAAGGACAAGCCAGGCGTCTACACGAGAGTCTCACACTTCTTACCCTGGATCCGC

1201     1210      1220      1230      1240      1250      1260      1270      1280
 AGTCACACCAAGGAAGAGAATGGCCTGGCCCTCTGAGGGTCCCCAGGGAGGAAACGGGCACCACCCGCTTTCTTGCTGGT
                                 Stop

1281     1290      1300      1310      1320      1330
 TGTCATTTTTGCAGTAGAGTCATCTCCATCAGCTGTAAGAAGAGACTGGGAAGAT .... 3'
```

(a) 5'-non-coding region and signal sequence:

As apparent from the above-mentioned Sequence I and Sequence III, the 5'-non-coding region, at least comprising 79 bases exists in the upstream of ATG (Met) which is an initiation codon for the synthesis of proteins. On the other hand, the known amino acid sequence of human urinary (Sequence II) does not contain the first 20 amino acids which start from Met (ATG). Therefore, this amino acid sequence was confirmed to be a signal peptide which is indispensable for the extracellular secretion of the urokinase molecule. Further, the amino acid sequence which starts from the 2lst amino acid (Ser) was found to correspond to the A-chain amino acid sequence of human urinary urokinase.

Hence, it is apparent that the cDNA obtained in the present example is just cDNA (pre-UK cDNA) which encodes the urokinase precursor (or prepro-urokinase, which is hereinafter referred to as "pre-UK"), which has the signal peptide comprising 20 amino acids.

(b) Cleavage of urokinase precursor:

Urokinase obtained from human urine has a double-stranded structure comprising A-and B-chains. It is believed that the urokinase precursor molecule (pro-UK) is first synthetized, and after the extracellular secretion of the pro-UK, the urokinase is secondarily cleaved into double-stranded chains because of the action of a protease or the like. This was confirmed by the sequencing of the cDNA of pUKl.

In Sequence II, Lys exists, following the amino acid sequence of the carboxyl-terminal of the A-chain of urokinase obtained from human urine (l57th amino acid, Phe, in Sequence II), and in addition, the B-chain has an amino acid sequence of Ile-Ile-Gly-Gly-. . . in the amino-terminal. This means that the urokinase obtained from human urine is first synthesized in the form of a single-chain pro-urokinase where the A-and B-chains are bonded to each other via Lys, that the urokinase can be cleaved in the Arg-Phe-Lys-Ile-Ile portion, and that Lys is lost after the cleavage.

(c) Carboxyl-terminal and 3'-non-coding region of cDNA

A part of the 5'-non-coding region, the coding region and the 3'-non-coding region of the cDNA obtained by the process of the present invention is shown in Sequence I and Sequence III. As shown in Sequence I and Sequence III, the amino acid sequence which corresponds to the carboxyl-terminal of human urinary high molecular weight-type urokinase, or Gly-Leu-Ala-Leu, is read out in this base sequence, and TGA, which is the termination codon, exists in downstream thereof. Accordingly, the carboxyl-terminal of the high molecular weight-type urokinase obtained from human urine and that of the cDNA as obtained by the process of the present invention are the same.

On the other hand, the DNA sequence in Sequence III does not contain any poly(A)-sequence, which is characteristic in the 3'-terminal of mRNA of eukaryotes. Therefore, the 3'-non-coding region is considered to be far longer than that as mentioned in the above.

(l3) Formation of plasmid containing urokinase complete coding region:

pUKl and pUK4 were digested with restriction enzymes Hind III and BglII. The digestion with Hind III and Bgl II was carried out at 37°C for l to 2 hours. A fragment of about 5.3 kb was isolated from pUKl, and a fragment of about l.3 kb was isolated from pUK4. Next, these fragments were ligated for 2 hours at 20°C in $T_4$ DNA ligase reaction buffer (comprising 66mM-Tris-HCl (pH 7.6), 66mM $MgCl_2$, l0mM dithiothreitol and 0.5mM ATP) and l0 units of $T_4$ DNA ligase. The thus obtained plasmid was named pUK33. The above-mentioned steps are shown in Fig. 3. In the Fig. 3, E means EcoR I, H means Hind III and B means BamH I.

(l4)Production of urokinase in established cells of human kidney:

(a) Formation of expression vector in cells:

$pcDV_1$ and $pL_1$ (see Fig. 4) were used as starting material plasmids, (see Okayama and Berg Molec. and Cell. Biol., 3, 280-289 (l983)), and pSV-$G_1$ (see Fig. 5) was formed therefrom. $pcDV_1$ and $pL_1$ are hybrid plasmids of pBR322 DNA and SV40 DNA, which were obtained from PL. Biochemicals (Pharmacia) Co. In order to facilitate the insertion of the uorkinase-coding cDNA into these plasmids, the Hind III-Pst I fragment of $pL_1$ was inserted into the Hind III-Kpn I site of $pcDV_1$ to form pSV-$G_1$.

More specifically, 4 $\mu$g of $pL_1$ was digested with Pst I under the reaction conditions of Table 5 above, and then, the protruding 3′-terminal was blunt-ended with $T_4$ DNA polymerase. In this reaction for the conversion of the 3′-terminal to the blunt one, the reaction mixture (comprising 4 $\mu$g of $pL_1$, 8 $\mu$l of $T_4$ polymerase buffer (l0X), 4.0 $\mu$l of 2mM dNTs solution and 76 $\mu$l of $dH_2O$) was heated at 65°C for 3 minutes and then rapidly cooled. Thereafter, 4 $\mu$l (l0U) of $T_4$ DNA polymerase was added thereto whereby the final amount of the reaction solution was made to be 80 $\mu$l, and the reaction was carried out at 37°C for 5 minutes. After completion of the reaction, 8 $\mu$l of 0.25M EDTA (pH 8.0) and 80 $\mu$l of $dH_2O$ were added to the reaction mixture and the resulting mixture was extracted once with phenol. The aqueous layer was recovered and ethanol was added thereto obtain DNA precipitate as described above. The DNA obtained was dried under reduced pressure and used for the following Kpn I linker ligation. In the ligation reaction, the reaction mixture (comprising 4 $\mu$g of dried DNA, 2 $\mu$l (2 $\mu$g) of 5′-P Kpn I linker, 2.0 $\mu$l of ligase buffer -(l0X) and l8 $\mu$l of $dH_2O$) was heated at 65°C for 3 minutes and then gradually cooled. Next, 2 $\mu$l (5U) of $T_4$-DNA ligase was added thereto and reacted overnight at l6°C. After completion of the reaction, the reaction mixture was digested with Kpn I and then with Hind III, and thereafter, the DNA was subjected to 5% (w/v) polyacrylamide gel electrophoresis (PAGE) to recover a DNA fragment of about 600 bp.

$pcDV_1$ was also digested with Hind III and Kpn I, and a DNA fragment of about 2.7 kbp was prepared with l% (w/v) agarose gel. l00 ng of the fragment was used for ligation with the above-mentioned DNA fragment of about 600 bp derived from $pL_1$ In this ligation reaction, 2 $\mu$l of $T_4$-DNA ligase (5.6U) was added to the reaction mixture (l50 ng of the $pL_1$ DNA fragment, l00 ng of the $pcDV_1$ DNA fragment, l.0 $\mu$l of $T_4$ DNA ligase buffer (l0X) and 8 $\mu$l of $dH_2O$) and reacted overnight at l6°C. Half of the reaction solution was used for the transformation of E. coli HBl0l.

From among the obtained transformants, l2 colonies were used for the preparation of plasmid DNA, and the resulting DNA was digested with a variety of restriction enzymes for the investigation of the constitution thereof. As a result, it was confirmed that all these DNAs contained the desired plasmid. Therefore, a large amount of plasmid DNA was prepared from one clone of the colonies to obtain pSV-$G_1$. The restriction enzyme map of pSV-$G_1$ was prepared (as shown in Fig. 5), and the DNA sequence of the Sal I-Bcl I DNA fragment was obtained (as shown in the following Sequence IV). As a result, it was confirmed that pSV-$G_1$ had a sequence containing an early enhancer/promoter element and a late 5′-splicing junction of SV-40 DNA upstream with a Kpn I cloning site and late poly (A) addition signal downstream.

Sequence IV: DNA-sequence of the Sal I-Bcl I DNA fragment of $pSV-G_1$

```
                                          5' GTCGAC AATTCTCATG TTTGACAGCT TATCATCGAT
                                             Sal I                              Cla I

AAGCTTGGCT GTGGAATGTG TGTCAGTTAG GGTGTGGAAA GTCCCCAGGC TCCCCAGCAG GCAGAAGTAT GCAAAGCATG CATCTCAATT AGTCAGCAAC
Hind III                                                   72bp repeat II

                                          21bp repeat III                          21bp repeat II
CAGGTGTGGA AAGTCCCCAG GCTCCCCAGC AGGCAGAAGT ATGCAAAGCA TGCATCTCAA TTAGTCAGCA ACCATAGTCC CGCCCCTAAC TCCGCCCATC
                      72bp repeat I

                      21bp repeat I                 17bp-AT        (capping site)     early mRNA  (in vivo)
CCGCCCCTAA CTCCGCCCAG TTCCGCCCAT TCTCCGCCCC ATGGCTGACT AATTTTTTTT ATTTATGCAG AGGCCGAGGC GGCCTCGGCC TCTGAGCTAT
                                                             TATA-BOX
                                                                                    16s/19s donor site
TCCAGAAGTA GTGAGGAGGC TTTTTTGGAG GCCTAGGCTT TTGCAAAAAG CTCCTCGAGG AACTGAAAAA CCAGAAAGTT AACTGGTAAG TTTAGTCTTT
                                                       Xho I linker               Hpa I

               19s acceptor site                                                   16s acceptor site
TTGTCTTTTA TTTCAGGTCC CGGATCCGGT GGTGGTGCAA ATCAAAGAAC TCGTCCTCAG TGGATGTTGC CTTTACTTCT AGGCCTGTAC CGAAGTGTTA
                  BamH I linker

CTTCTGCTCT AAAAGCTGCC GGTACCTTCT GAGGCGGAAA GAACCAGCCG GATCCTCGAG TGATAAGATA CATTGATGAG TTTGGACAAA CCACAACTAG
                      Kpn I                            BamH I/Xho I
                                                                                       Hpa I
AATGCAGTGA AAAAAATGCT TTATTTGTGA AATTTGTGAT GCTATTGCTT TATTTGTAAC CATTATAAGC TGCAATAAAC AAGTTAACAA CAACAATTGC
                                                                                 POLY (A) site

AATCATTTTA TGTTTCAGGT TCAGGGGGAG GTGTGGGAGG TTTTTTAAAG CAAGTAAAAC CTCTACAAAT GTGGTATGGC TGATTATGAT CA 3'
                                                                                           Bcl I
```

(b) Insertion of urokinase-DNA into vector:

pUK33 as obtained in the above-mentioned step (13) was partially digested with Pst I, and a 1.7 Kbp fragment was isolated therefrom and inserted into pSV-$G_1$. For the isolation of the 1.7 Kbp fragment, the reaction mixture (10 µg of pUK33, 50 µl of Pst I-buffer (10X), 10 µl of Pst I (60U) and $dH_2O$, the final amount of the solution being 500 µl) was kept at 37°C. After 10 minutes, 15 minutes and 20 minutes from the beginning of the reaction at 37°C, about 150 µl was removed in each case and the samples were heat-treated at 65°C for 5 minutes. After the heat-treatment, all of the thus heat-treated samples were combined and subjected to electrophoresis on a 1% (w/v) agarose gel, whereby about 10 µg of the desired 1.7 Kbp fragment was recovered. About 5 µg of the fragment from among the thus recovered of about 1.7 Kbp fragment was treated with $T_4$ DNA-polymerase whereby the cleaved terminal was blunt-ended, and this was ligated with a Kpn I-linker. The resulting product was ligated with Kpn I-digested pSV-$G_1$ for the transformation of E. coli HB101, to obtain pSV-$G_1$-preUK. (See Fig. 6.)

As shown in Fig. 7, pSV-$G_1$-preUK contains the 5′-non-coding region, the signal sequence coding region and the 3′-non-coding region of pre-UK-cDNA inserted at the Kpn I site of pSU-$G_1$. Accordingly, when the DNA is introduced into cells such as established cells of human kidney, the secretion of the human-pro-urokinase is possible.

(c) Preparation of dominant selection marker-containing plasmid-DNA:

pSV-$G_1$-$Neo^r$ which is a plasmid to be used as a dominant selection marker in the transformation of cells was prepared as follows:

Plasmid pNEO which carried Tn5-derived neomycin-resistant gene (Southern, P.J. and P. Berg, J. Molec. and Applied Genet., 1, 327-341 (1982)) (manufactured by PL-Biochemical Co.) was digested with BamH I and Hind III and then, the 1.5 Kbp neomycin-resistant gene-containing DNA fragment was recovered therefrom. The recovered DNA was treated with E. coli DNA polymerase I, Klenow fragment, in the presence of 4 dNTP's whereby the cleaved terminal was blunt-ended, and then, reacted with $T_4$ DNA ligase whereby a Kpn I-linker was added to the end thereof in the same manner as described above.

After the Kpn I-linker ligation, the DNA fragment was completely digested with Kpn I (6U), and thus, the desired linker-added neomycin-resistant gene of about 1.5 Kbp was recovered.

From among the thus recovered DNA fragment, 15 ng of the fragment was ligated with 20 ng of Kpn I-cleaved pSV-$G_1$ in the same manner as described above for the transformation of E. coli HB 101, to obtain the desired plasmid pSV-$G_1$-$Neo^r$ (See Fig. 6.)

(d) Preparation of recipient cells:

Established cells obtained by subcultivation of the primary culture from human fetal kidney cells, were used as the recipient. The cells were used for DNA-transfection and these were collected by trypsin-EDTA treatment and then suspended in a culture medium (5% (v/v) FCS-containing Dulbecco's MEM medium). The cells was inoculated in a Falcon # 3003 (100mm)-culture dish in an amount of 7 x $10^5$ cells/10ml/100mm-dish. The cells were incubated in a $CO_2$ incubator (containing 5% (v/v) $CO_2$ and 95% (v/v) air) for 24 hours and then used for transformation. Under these conditions, the urokinase-productivity of the established cells of human-kidney origin was 26.51 U/ml/day (average of five samples). (See Table-6.)

(e) Transformation:

A DNA-$CaPO_4$ co-precipitation technique was performed to transform the recipient cell in accordance with the method of Osada, et al., Protein, Nucleic Acid, Enzyme, 28, (14), 1569-1581, 1983. Mixture of plasmid DNA (pSV-$G_1$-PreUK and pSV-$G_1$-Neo $^r$ with the mixture ratio of 100:1) was transfected to the cells in an amount of 2 to 4 µg(DNA)/dish. The cells were incubated for 6 to 9 hours, and the culture medium was changed by a fresh one and the incubation was continued further for 48 hours or more.

(f) Selection of transformed cells:

The urokinase gene itself is not selectable, thus the G-4l8-resistance gene derived from Tn 5 was used as the dominant selection marker (above-mentioned pSV-$G_1$-Neo). The cells were included in G-4l8-containing culture medium (400 µg/ml) in accordance with the method of Southern (J. Molec. and Applied Genet., I , 327-34l, l982), whereby the selection of the transfected cells was carried out. The G-4l8 as used was Geneticin (registered trademark for a product of Gibco Co.).

The culture medium was changed by a fresh one on every 5th day. 50 colonies resistant to G-4l8 were obtained after about l4 days. These colonies were further grown in 5 (v/v)-FCS-containing Dulbecco's MEM medium. The thus grown colonies were screened and two colonies which were considered to have a higher urokinase-productivity were selected out. The culture supernatant of these colonies was isolated, and the increment of the urokinase-productivity because of the transfection with the urokinase-cDNA was investigated on the thus selected cells.

For the measurement of the activity, the cells were inoculated in an amount of 7 $\times$ $10^5$ cells/l0ml/25cm$^2$ flask and incubated for 3 days. Afterwards, the culture medium was changed for 5ml of a culture medium supplemented with l% (v/v) FCS (maintenance medium) and then incubated for further 24 hours. Thus, the urokinase activity in the culture fluid was measured and represented by the unit of IU/ml/day. The urokinase-productivity per cell was obtained from the amount of the urokinase produced in 24 hours and the average cell number used for the production of the amount of urokinase, and was represented by the unit of IU/cell/day.

Table 6

| Cells | Urokinase production | |
|---|---|---|
| | IU/ml/day | IU/cell/day ($\times 10^{-6}$) |
| HKG | 27 (n = 5) | 30 (n = 5) |
| WS52 - 11 | 59 (n = 3) | 59 (n = 3) |
| WS52 - 08 | 37 (n = 3) | 50 (n = 3) |

HKG: Established cells of human kidney origin used as recipient cells.
WS52-ll, WS52-08: Transformed cell lines.

(g) Property of human-urokinase as produced by transformed cells of human kidney origin:

The properties of the human-urokinase produced and secreted in the culture medium from the transformed cells were observed.

In the first place, the amount of the human-urokinase produced was determined. The transformed cells were inoculated at a cell density of 7 $\times$ $10^5$ cells/l0ml/25cm$^2$-flask and incubated therein for 3 days, and then the medium was changed for maintenance medium in an amount of 5ml/flask. After further incubation for 24 hours, the plasminogen activator activity in the supernatant was measured. (See Table 6 above.)

As a result of the measurement of the urokinase activity in a fibrin-plate method (by Astrup T., et al., Arch. Biochem. Biophys., 40, 346-35l (l952)) using human urinary urokinase as reference standard, it was confirmed that the urokinase-productivity of the transformed cells was increased at least twice that of the non-transformed u. kinase-producing strains, as shown in Table 6. In addition, the activity was specifically neutralized with an anti-human urokinase-antibody.

Next, the molecular weight of the human-urokinase produced by these clones was measured as follows:

The culture fluid as cultured in the maintenance medium for 24 hours was subjected to electrophoresis through l2.5% (v/v) SDS PAGE, and the protein band was blotted electrophoretically to a nitrocellulose filter in 25mM Tris HCl and l92mM glycine (pH 8.3)/20% (v/v) methanol and then was subjected to Western Blotting in accordance with the method by Towbin et al, Proc. Natl. Acad. Aci., USA, 76, 4350-, 1979.

The filter was thereafter blocked in gelatin-containing 20mM Tris HCl (pH 7.5)/500mM NaCl, at room temperature for 60 minutes and incubated at room temperature for 2 hours in a buffer containing anti-human urokinase, and then, the human urokinase band was detected by the use of an Immuno-Blot™ Assay System made by Bio-Rad Co. with HRP (horse-radish peroxidase). As a primary antibody, an affinity-purified anti-human-urokinase rabbit-IgG was used.

In the case of the sample which had been reduced with 2-mercapto-ethanol, only a band of urokinase with a molecular weight of 54K was detected. Regarding the 54K-band, the present sample was the same as the other referential sample of human natural-type single-chain pro-UK (supernatant of cultured human fetal kidney cells), which had been subjected to the same electrophoresis, in terms of both the molecular weight and the reactivity to the anti-human-urokinase IgG, and the two samples could not be distinguished from each other. (See Fig. 8). This result is considered to prove that the transformed cells were made to express the human urokinase-cDNA, which had been introduced thereinto by the DNA-transfection, in addition to the urokinase gene which existed in the original cells, whereby the productivity of the glycosylated single-chain pro-UK having a molecular weight of 54K.

Next, the two samples were subjected to column-chromatography with Con A-Sepharose 4B made by Pharmarcia Co., whereupon human-urokinase as synthesized and secreted by each clone was investigated.

In the chromatography experiment, the clone was cultured until the culture became confluent and then washed twice with Dulbecco's PBS(+) made by Nisshui co. Afterwards, the clone was cultured in 5ml of methionine-free Dulbecco's MEM medium supplemented with l% (v/v) FCS, which had been dialyzed against PBS(+) together with ($^{35}$S)-methionine for 20 hours.

The ($^{35}$S)-labeled culture fluid was applied to 5ml of an anti-human-urokinase monoclonal-antibody column. The column was washed with a 0.04M sodium phosphate buffer (pH 8.0)/0.03M NaCl, and then, the $^{35}$S-labeled human urokinase was eluted with glycine-HCl buffer (pH 2.5). The thus recovered $^{35}$S-labeled human-urokinase was neutralized with lM Tris-HCl and then subjected to dialysis against 20mM sodium phosphate buffer (pH 7.4)/l50mM NaCl and thereafter applied to ConA-Sepharose 4B column made by Pharmarcia Co. which had been equilibrated with the same buffer.

After washed with the buffer, the column was subjected to elution with 0.lM methyl-α-D-mannoside containing sodium phosphate buffer (pH 7.4). As a result, almost all the radio-active urokinase adsorbed to the Con A-Sepharose was specifically eluted by methyl-α-D-mannoside.

The above result proves that the human-pro-urokinase-cDNA introduced into the established cells of human kidney origin is normally transcribed therein whereby a glycoprotein having the same molecular weight and the same monoclonal antibody-reactivity as those of the corresponding natural-type can be synthesized in the transformed cells.

While the invention has been described in detail and with reference to specific embodiments thereof, it will be apparent to one skilled in the art that various changes and modifications can be made therein without departing from the spirit and scope thereof.

## Claims

l. A process for producing physiologically active substance comprising the steps of (l) transforming established cells of human kidney origin with a vector DNA having therein a DNA sequence which encodes a human physiologically active substance, (2) growing the resulting transformed cells, and (3) collecting said physiologically active substance which has been secreted from said transformed cells.

2. The process for producing physiologically active substances as claimed in claim l, wherein the physiologically active substance is a plasminogen activator selected from the group consisting of tPA, urokinase and single-chain prourokinase.

3. The process for producing physiologically active substances as claimed in Claim l, wherein said vector DNA having a DNA sequence which encodes a human physiologically active substance is pSV-$G_1$-PreUK.

4. The process for producing physiologically active substances as claimed in Claim l, wherein said transformed cell is selected from the group consisting of WS52-ll and WS52-08.

5. The process for procuding physiologically active substances as claimed in Claim l, wherein said physiologically active substance is urokinase.

FIG. 1

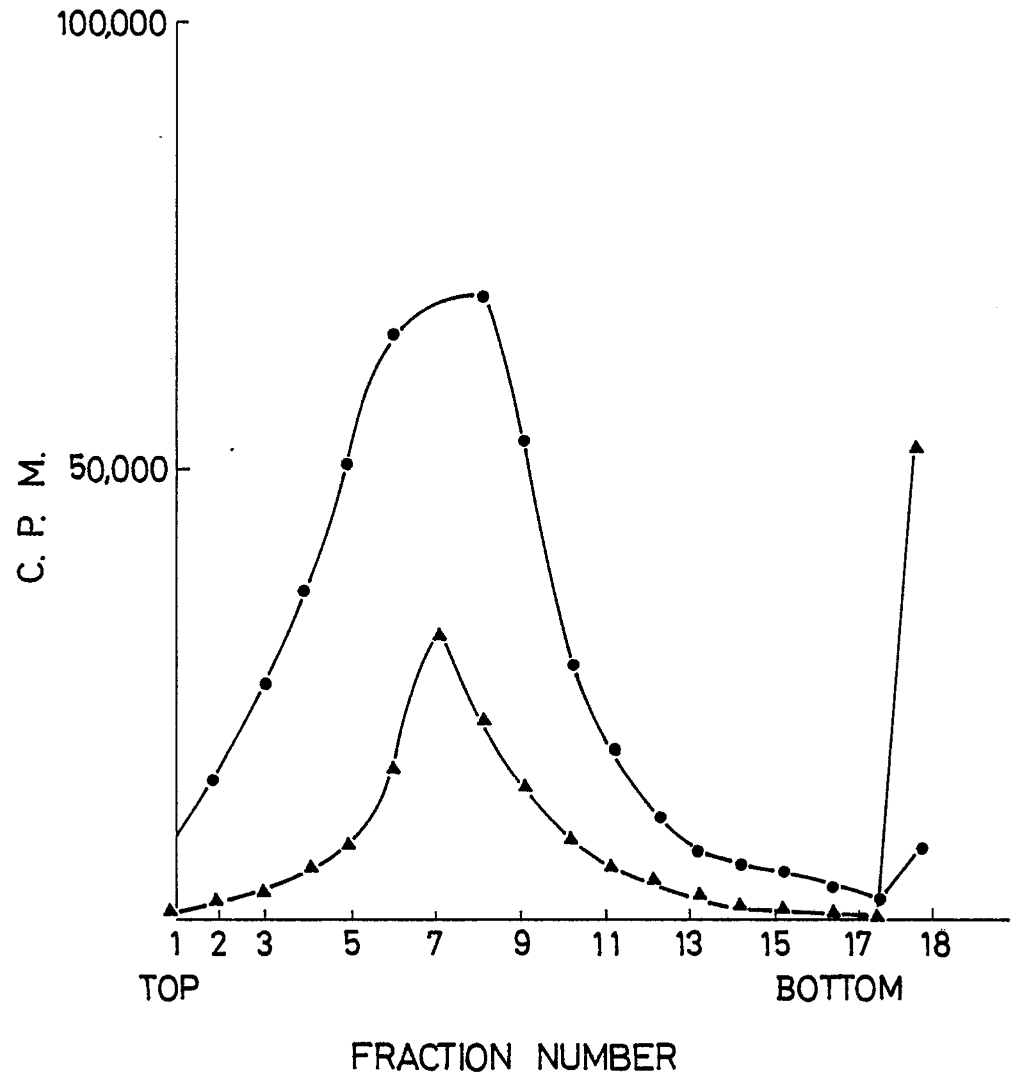
100,000
50,000
C. P. M.
1
2
3
5
7
9
11
13
15
17
18
TOP
BOTTOM
FRACTION NUMBER

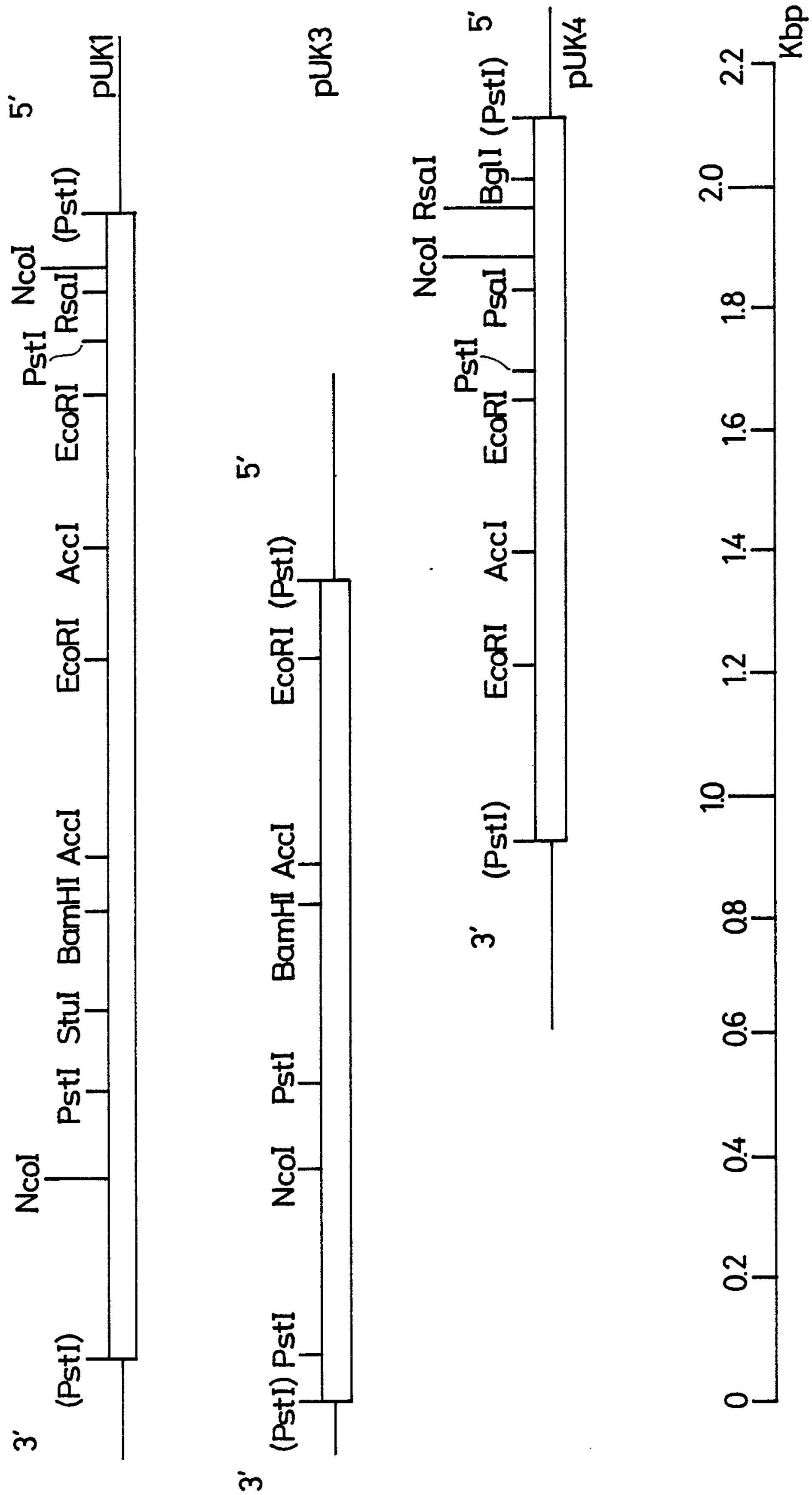

pUK1
5'
3'
(PstI) NcoI RsaI PstI EcoRI AccI EcoRI AccI BamHI StuI PstI NcoI (PstI)
pUK3
5'
3'
EcoRI (PstI) EcoRI AccI BamHI PstI NcoI PstI (PstI)
pUK4
5'
3'
BglI (PstI) NcoI RsaI PsaI PstI EcoRI AccI EcoRI (PstI)
0
0.2
0.4
0.6
0.8
1.0
1.2
1.4
1.6
1.8
2.0
2.2
Kbp


FIG. 2

FIG. 3

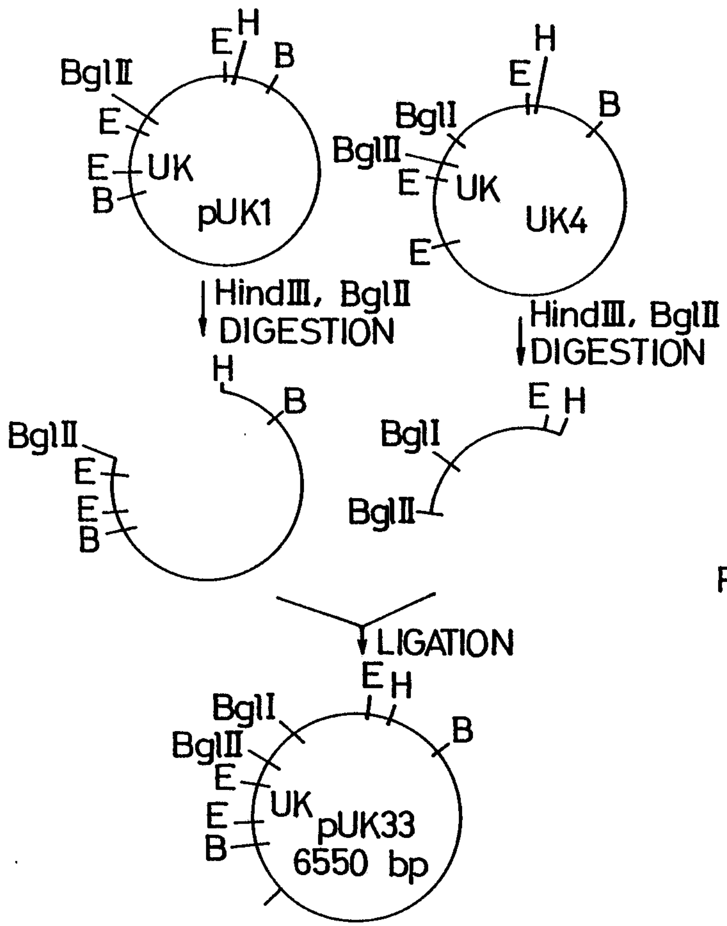

pUK1
UK
BglII
E
E
B
E
H
B
UK4
BglI
BglII
HindIII, BglII DIGESTION
HindIII, BglII DIGESTION
LIGATION
pUK33
6550 bp


FIG. 4

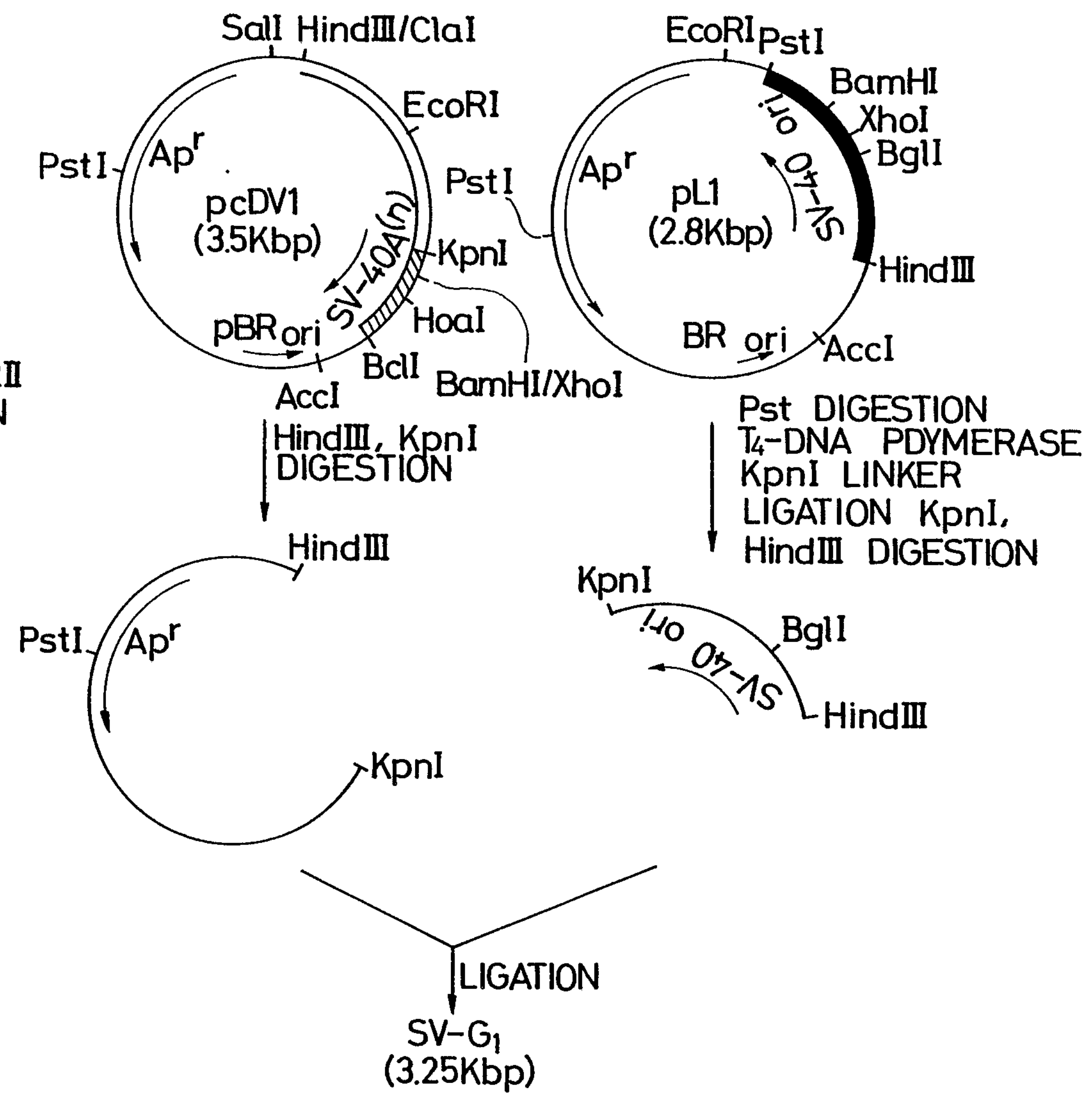

SalI HindIII/ClaI
EcoRI
PstI
Apr
pcDV1
(3.5Kbp)
SV-40A(n)
KpnI
HoaI
BclI
BamHI/XhoI
pBR ori
AccI
EcoRI PstI
BamHI
XhoI
BglI
HindIII
SV-40 ori
pL1
(2.8Kbp)
BR ori
AccI
HindIII, KpnI DIGESTION
Pst DIGESTION
T4-DNA PDYMERASE
KpnI LINKER
LIGATION KpnI,
HindIII DIGESTION
LIGATION
SV-G1
(3.25Kbp)

FIG. 5

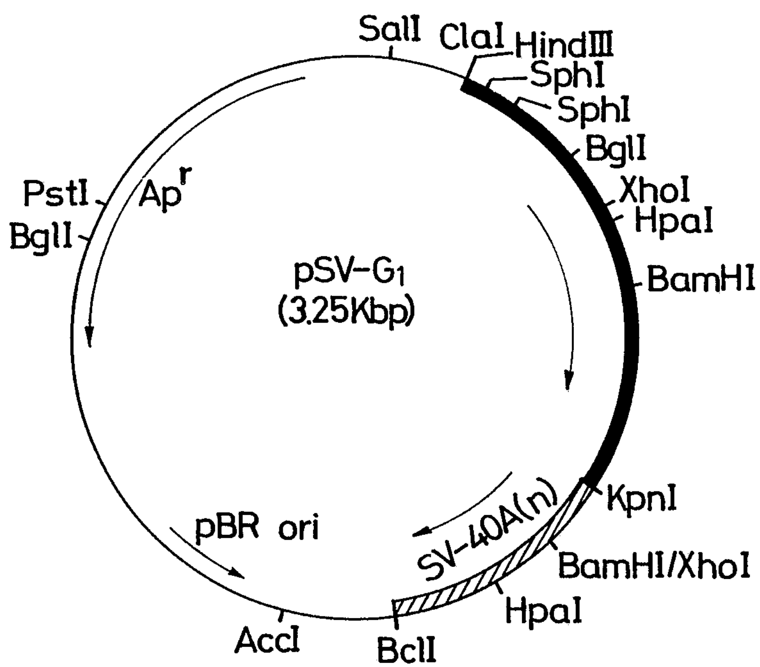
SalI
ClaI
HindIII
SphI
SphI
BglI
XhoI
HpaI
BamHI
KpnI
BamHI/XhoI
HpaI
BclI
AccI
pBR ori
SV-40A(n)
PstI
BglI
Ap^r
pSV-G1
(3.25Kbp)

FIG. 6

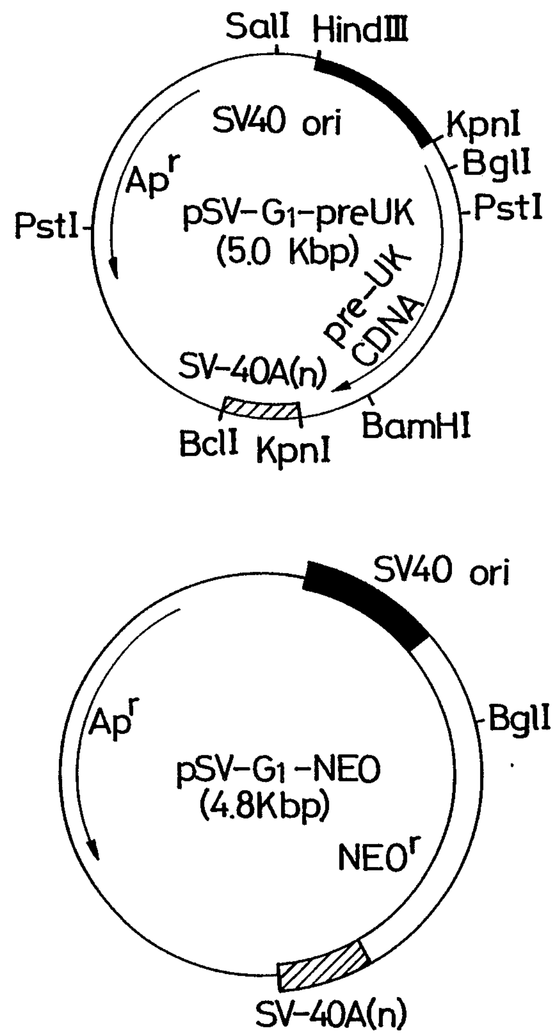
SalI
HindIII
KpnI
BglI
PstI
BamHI
KpnI
BclI
SV-40A(n)
pre-UK
CDNA
PstI
Ap^r
SV40 ori
pSV-G1-preUK
(5.0 Kbp)
SV40 ori
BglI
NEO^r
SV-40A(n)
Ap^r
pSV-G1-NEO
(4.8Kbp)

FIG. 7

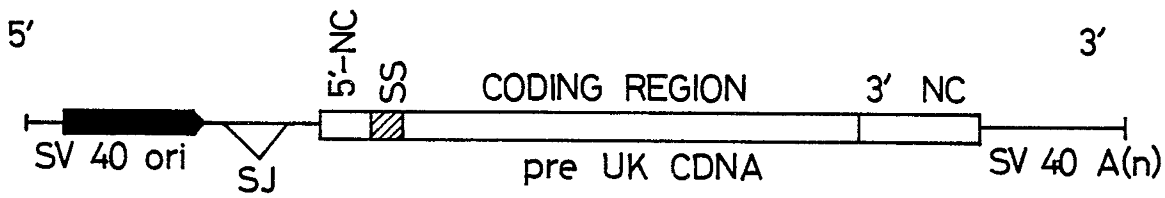
5'
3'
5'-NC
SS
CODING REGION
3' NC
SV 40 ori
SJ
pre UK CDNA
SV 40 A(n)

FIG. 8

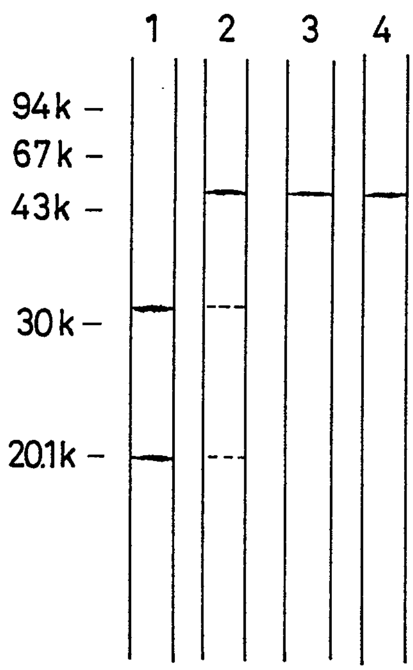
1
2
3
4
94k
67k
43k
30k
20.1k